# EUROPEAN PATENT APPLICATION

(11) **EP 1 464 624 A1**
(43) Date of publication of application: **06.10.2004**
(21) Application number: 04005459.5
(22) Date of filing: 08.03.2004
(51) Int. Cl.: C02F 3/10, B65D 41/04, C12N 11/00

(54) **Carrier for biofilm made of screw caps to be used in wastewater purification plants**

(30) Priority: 18.03.2003 IT PD20030055
(71) Applicant: ACQUA MINERALE SAN BENEDETTO S.p.A., I-30037 Scorze' (Prov. of Venezia) (IT)
(72) Inventor: Sonego, Roberto, 31020 San Pietro di Filetto (Treviso) (IT)
(74) Representative: Modiano, Guido, Dr.-Ing.

(57) **Abstract**

A carrier (10) for biofilm to be used in wastewater purification plants, comprising a cup-shaped body (11) that is formed by a substantially circular bottom (12), by a perimetric wall (13) that has substantially a cylindrical symmetry and by an opening (14) that is arranged opposite the bottom (12) for the access of the wastewater; a helical wing (16) runs along the inner side (15) of the perimetric wall (13) and accordingly has a portion of surface (17) that is undercut with respect to the opening (14). Advantageously, the carrier (10) is constituted by an ordinary screw cap for bottles, which is made of plastics, or by a reject from the production of screw caps for bottles.

## Description

The present invention relates to a carrier for biofilm to be used in wastewater purification plants.

Currently, in the biological purification of water from industrial processes of various kinds, the water is passed through one or more reactors that contain microorganisms that allow to purify the water, for example by removing carbon and nitrogen.

The structure of these reactors is often constituted by large tanks through which the water to be purified passes.

Depending on the type of purification process, amounts of air may be injected into the tanks (aerobic purification).

The purification capacity of this type of process is not high, since the microorganisms that are responsible for purification are expelled from the tanks when they are still too young to ensure optimum purification and excessively scattered to produce good clarification.

To obviate these drawbacks, said microorganisms are made to grow on a very large number of carriers immersed in said tanks.

The surface of said carriers allows the accelerated growth of a biofilm formed by said microorganisms.

The carriers inside said tanks are generally made of a polymeric resin, such that their density allows them to remain suspended in the tanks.

The application of said carriers leads to a significant increase in the specific volumetric purification capacity of the plants in which they are used.

This increase is a function of the availability of aeration of the tanks and of the number of carriers used.

Currently known carriers are constituted by an extruded central body (with a cross-section shaped like a circle or square or triangle et cetera), formed by perimetric walls and by opposite openings for the passage of water from one end to the other.

On the perimetric walls, both externally and internally, there are wings that increase, in practice by corrugating it, the biofilm adhesion surface.

The shape of the walls and wings is such as to try to protect the biofilm as much as possible against damage due to impacts between the various carriers and against the movement of the water that tends to tear it away.

The water in fact is often moving vortically both due to the inflow and outflow motion of the water inside the tanks and often due to the presence of aerators, agitators and mixers.

It should be noted that the cost for the use of said carriers is high.

In fact, although each individual carrier has very small dimensions and has an apparently low cost (approximately 0.0005 euro), in view of the extremely large number of carriers generally required in a purification plant (on the order of hundreds of thousands, if not millions), the overall cost is very high.

The aim of the present invention is to provide a carrier for biofilm to be used in wastewater purification plants that allows to achieve overall cost reductions in the use of said carriers and at the same time ensures optimum growth for said biofilms.

Within this aim, an object of the present invention is to provide a carrier for biofilm to be used in wastewater purification plants that is provided with growth surfaces for said biofilms that are protected from the outside.

Another object of the present invention is to provide a carrier for biofilm to be used in wastewater purification plants that can be manufactured with known systems and technologies.

This aim and these and other objects that will become better apparent hereinafter are achieved by a carrier for biofilm to be used in wastewater purification plants, characterized in that it comprises a cup-shaped body that is formed by a substantially circular bottom, by a perimetric wall that has substantially a cylindrical symmetry and by an opening that is arranged opposite said bottom for the access of the wastewater, at least one wing that has a portion of surface that is undercut with respect to said opening protruding along the inner side of said perimetric wall.

Advantageously, the invention uses screw caps for bottles, made of plastic material, as carriers for biofilm in wastewater purification plants.

Conveniently, the invention also provides for the use of a cap rejected from the production of screw caps for bottles made of plastics as a carrier for biofilm to be used in wastewater purification plants.

Further characteristics and advantages of the present invention will become better apparent hereinafter from the following detailed description of a preferred but not exclusive embodiment thereof, illustrated by way of nonlimiting example in the accompanying drawings, wherein:

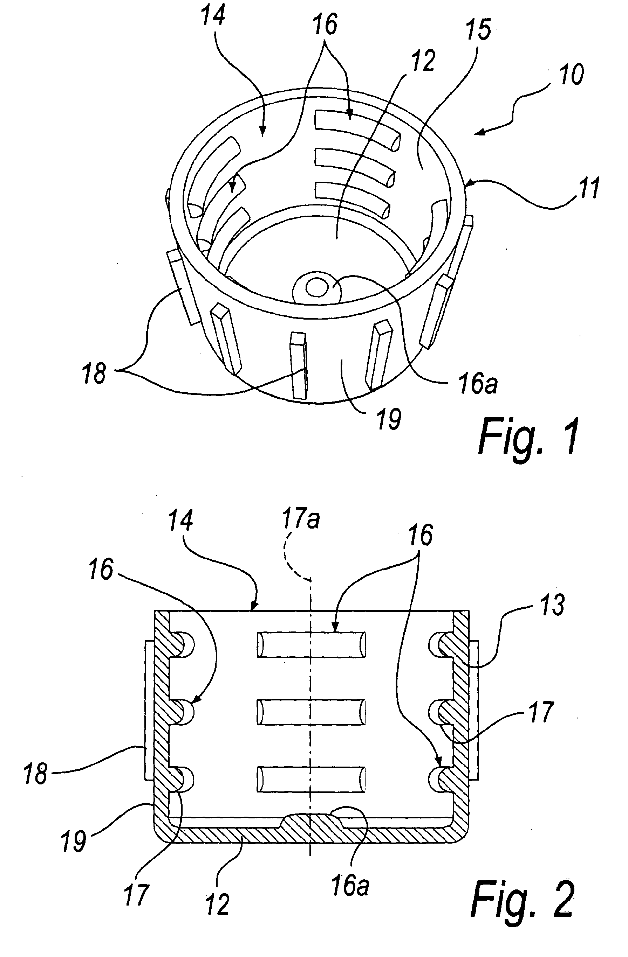

Figure 1 is a perspective view of a carrier for biofilm according to the invention;
Figure 2 is a sectional view of the carrier of Figure 1;
Figure 3 is a perspective view of a screw cap for bottles that constitutes a carrier according to the invention;
Figure 4 is a sectional view of the cap of Figure 3.

With reference to the figures, a carrier for biofilm to be used in wastewater purification plants according to the invention is generally designated by the reference numeral 10.

The carrier 10, preferably made of plastics of suitable density, comprises a cup-shaped body 11 that is formed by a substantially disk-like bottom 12, by a perimetric wall 13 that has a substantially cylindrical symmetry, and by an opening 14 that is arranged opposite the bottom 12 for the access of the wastewater.

Wings 16 protrude along an inner side 15 of the perimetric wall 13 with a convex surface region in an annular pattern; each wing has a surface portion 17 that is undercut with respect to the opening 14 and with respect to the convex surface region of the wing.

In particular, the wings 16 are arranged in an annular pattern on three different planes that are perpendicular to an axis 17a of said carrier, thus forming spaces between said wings that are in a shadow zone with respect to the opening 14 and the bottom 12.

In an alternative embodiment, the wings 16 can be arranged on the inner side 15 along a helical arrangement and optionally extend continuously so as to form a single wing.

A protrusion 16a with an annular contour protrudes outward from the bottom 12.

Said cup-shaped body further comprises longitudinal ribs 18 that are mutually equidistant and protrude from an outer side 19 of the perimetric wall 13.

Advantageously, the support 10 can be constituted by an ordinary screw cap for bottles, made of plastics, or by a reject cap originating from the production of screw caps for bottles.

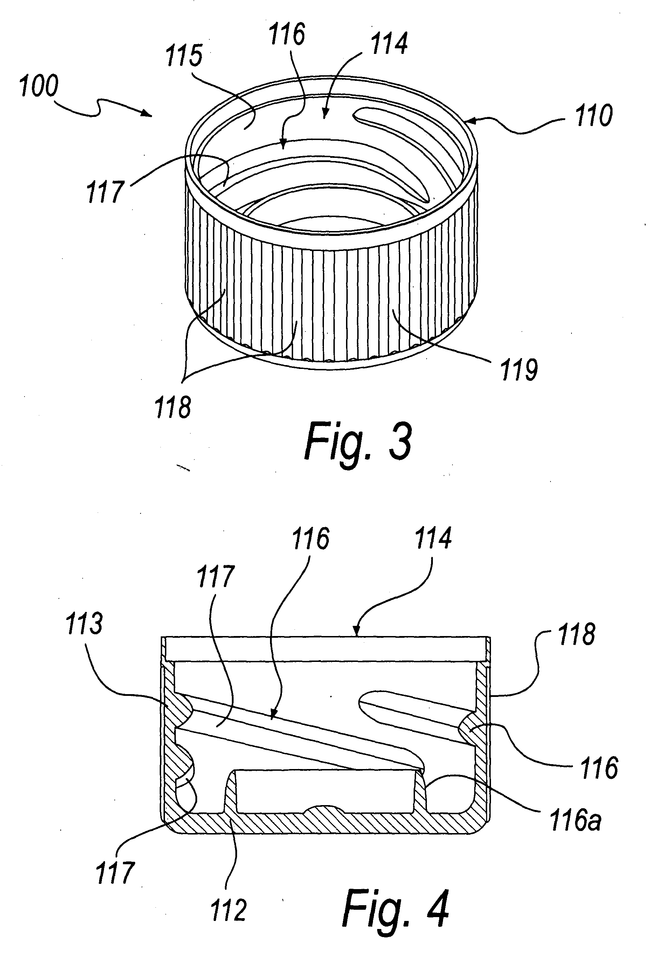

With reference to Figures 3 and 4, therefore, a cap that can be used as a carrier is designated by the reference numeral 100.

The cap 100, made of plastics, comprises a cup-shaped body 110 that is formed by a substantially circular bottom 112, by a perimetric wall 113 that substantially has a cylindrical symmetry, and by an opening 114 that lies opposite the bottom 112 for the access of the wastewater.

A helical wing 116 protrudes along an inner side 115 of the perimetric wall 113 with a convex surface region and accordingly has a surface portion 117 that is undercut with respect to the opening 114 and to the convex surface region of the wing.

A protrusion 116a protrudes outward from the bottom 112 and has an annular contour.

The cap 100 further comprises longitudinal ribs 118 that are mutually equidistant and protrude from an outer side 119 of the perimetric wall 113.

Experimental tests have in fact shown that these carriers constituted by screw caps or rejects of screw caps have optimum adhesion of the biofilm and a consequent optimum purifying action with respect to wastewater, particularly with respect to wastewater from the industrial processing of beverages.

In practice it has been found that the invention thus described solves the problems noted in known types of biofilm carrier to be used in wastewater purification plants; in particular, the present invention provides a carrier for biofilm to be used in wastewater purification plants that allows to reduce the overall operating costs of said carriers.

By using products of the waste of the production of screw caps, carriers for biofilms are in fact provided at zero cost.

Moreover, the present invention provides a carrier for biofilm to be used in wastewater purification plants that has surfaces for the growth of the biofilm that are sufficiently protected against impacts with other carriers.

The invention thus conceived is susceptible of numerous modifications and variations, all of which are within the scope of the appended claims; all the details may further be replaced with other technically equivalent elements.

In practice, the materials employed, so long as they are compatible with the specific use, as well as the dimensions, may be any according to requirements and to the state of the art.

The disclosures in Italian Patent Application No. PD2003A000055 from which this application claims priority are incorporated herein by reference.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A carrier for biofilm to be used in wastewater purification plants, **characterized in that** it comprises a cup-shaped body (11) that is formed by a substantially circular bottom (12), by a perimetric wall (13) that has substantially a cylindrical symmetry and by an opening (14) that is arranged opposite said bottom (12) for the access of the wastewater, at least one wing (16) that has a portion of surface (17) that is undercut with respect to said opening (14) protruding along the inner side (15) of said perimetric wall (13).

2. The carrier for biofilm according to claim 1, **characterized in that** it comprises at least one protrusion (16a) that protrudes from said bottom (12).

3. The carrier for biofilm according to claims 1 or 2, **characterized in that** said at least one wing (16) is at least partially helical.

4. The carrier for biofilm according to one or more of the preceding claims, **characterized in that** said cup-shaped body (11) comprises mutually equidistant longitudinal ribs (18) that protrude from the outer side (19) of said perimetric wall (13).

5. The carrier for biofilm according to one or more of the preceding claims, **characterized in that** it is made of plastics.

6. The use of screw caps for bottles made of plastics as carriers for biofilm in wastewater purification plants.

7. The use of reject caps from the production of screw caps for bottles made of plastics as carriers for biofilm in wastewater purification plants.
